# EUROPÄISCHE PATENTSCHRIFT

(11) **EP 0 704 207 B1**
(45) Veröffentlichungstag und Bekanntmachung des Hinweises auf die Patenterteilung: **25.07.2001**
(21) Anmeldenummer: 95113467.5
(22) Anmeldetag: 28.08.1995
(51) Int. Cl.: A61K 9/32, A61K 9/52, C08F 220/18

(54) **Thermoplastischer Kunststoff für darmsaftlösliche Arznei-Umhüllungen**
Thermoplastic resin for enteric pharmaceutical coating
Résine thermoplastique pour enrobages pharmaceutiques enterosolubles

(30) Priorität: 31.08.1994 DE 9414065 U
(43) Veröffentlichungstag der Anmeldung: 03.04.1996
(73) Patentinhaber: Röhm GmbH & Co. KG, 64293 Darmstadt (DE)
(72) Erfinder: Lehmann, Klaus Dr., D-64380 Rossdorf (DE); Höss, Werner, D-63150 Heusenstamm (DE)

(56) Entgegenhaltungen:
- EP-A- 0 143 935
- EP-A- 0 403 959
- GB-A- 2 091 203

## Beschreibung

Die Erfindung liegt auf dem Gebiet der Hilfsmittel zur Herstellung von darmsaftlöslichen Arznei-Umhüllungen. Eine wichtige Klasse von Hilfsmitteln für derartige Umhüllungen besteht aus Mischpolymerisaten von Acryl- und/oder Methacrylsäure mit niederen Alkylestern dieser Säuren. Sie werden in Form von Lösungen in organischen Lösungsmitteln oder als wäßrige Dispersionen bzw. Redispersionen zur Herstellung von Arzneiformumhüllungen eingesetzt. Die Verarbeitung dieser Lösungen und Dispersionen ist stets mit zeit- und energieaufwendigen Trocknungsschritten zur Verdunstung des eingesetzten Wassers oder Lösungsmittels verbunden. Zur Herstellung von Steckkapseln werden Schichten der Lösungen oder Dispersionen auf geeignet geformten Dornen eingetrocknet und abgezogen; vgl. DE 21 57 435, DE 35 24 337.

Es ist auch schon bekannt, Stärke durch Erhitzen mit Wasser zu plastifizieren und durch Spritzgießen zu Steckkapseln zu verarbeiten. Jedoch konnten Steckkapseln aus den erwähnten darmsaftlöslichen Acrylpolymeren bisher nicht durch rationelle thermische Formungsverfahren nicht hergestellt werden; vgl. L. Eith u.a., Drug Der.Ind.Pharm. 12 (11-13), S.2113-2126, 1986.

Weiterhin ist es bekannt, Arzneiformen in schweißbare Folien aus einem im Verdauungstrakt löslichen oder zerfallenden Material auf Basis von Gelatine einzuschließen und in dieser Form oral zu applizieren; vgl. WO 91/04017. Gemäß DE 35 25 767 werden organische Lösungen von pharmazeutischen Wirkstoffen und von Copolymerisaten von Methyl- und/oder Ethylestern der Acryl- und methacrylsäure mit niedrigen Erweichungstemperaturen eingetrocknet, der Rückstand pulverisiert und zu Tabletten verpreßt.

Aus der EP-B 143 935 sind thermoplastische Kunststoff-Formmassen auf Basis eines Mischpolymerisats aus 1 Mol Acryl- und/oder Methacrylsäure und 2,5 - 6 Mol mindestens eines Acrylats bekannt, die sich thermoplastisch zu Folien, Tiefziehteilen, Spritzgußteilen, Preß- oder Blasteilen verarbeiten lassen. Die Mischpolymerisate enthalten jedoch höchstens 15 Gew.-% an Carboxylgruppen, was zur Herstellung darmsaftlöslicher Arzneiform-Umhüllungen nicht ausreicht. Aus den bekannten Mischpolymerisaten werden alkalilösliche Folien für Hygiene- und Verpackungszwecke sowie Klebstoffe und Verpackungsteile hergestellt.

Die Erfinder haben festgestellt, daß die bekannten, aus wäßriger Dispersion oder organischer Lösung verarbeitbaren darmsaftlöslichen Acrylpolymeren, die aus 30 bis 50 Gew.-% Methacrylsäure und zum übrigen Teil aus Ethylacrylat oder Methylmethacrylat aufgebaut sind, nicht unzersetzt im thermoplastischen Zustand verarbeitbar sind. Sie beginnen bei Temperaturen oberhalb 120°C zu decarboxylieren und sind dann nicht mehr darmsaftlöslich.

Der Erfindung liegt die Aufgabe zugrunde, eine thermoplastische Kunststoff-Formmasse zur Verfügung zu stellen, die sich durch bekannte Thermoformungsverfahren zur Herstellung von darmsaftlöslichen Arzneiformen, beispielsweise zu Steckkapseln, verwenden läßt.

Es wurde gefunden, daß Mischpolymerisate aus
A) 16 bis 40 Gew.-% Acryl- und/oder Methacrylsäure,
B) 30 bis 80 Gew.-% Methylacrylat
C) 0 bis 40 Gew.-% anderen Alkylestern der Acryl-und/oder Methacrylsäure
für diesen Zweck geeignet sind. Daraus - gegebenenfalls unter Zusatz von üblichen Hilfsstoffen - hergestellte Arneimittelüberzüge sind im Magensaft bei pH 1 bis 2 sowie in Verdauungssäften oder Pufferlösungen mit pH-Werten ≤ 5 unlöslich, aber im Darmsaft bei pH-Werten von 5,5 bis 8 gut löslich. Sie lassen sich im thermoplastischen Zustand unzersetzt verarbeiten.

Steckkapseln bzw. Steckkapselhälften lassen sich - vorzugsweise mit Mehrfachwerkzeugen - durch das Spritzgießverfahren aus einer Schmelze des Mischpolymerisats bei Temperaturen von 140 bis 180°C herstellen. Das Spritzgießverfahren läßt sich auch anwenden, um vorgeformte Arzneikerne mit einem Polymerisatmantel zu umhüllen. Dabei werden die Kerne durch Hilfswerkzeuge, die vor der endgültigen Füllung des Formhohlraumes und vor dem Erstarren der Formmasse aus der Hohlform zurückgezogen werden, zentriert gehalten. Weitere Umhüllungsverfahren sind in WO 91/04017 beschrieben.

Aus einer Schmelze der erfindungsgemäßen Formmasse kann durch Extrusion und gegebenenfalls Glättung in einem Walzenglättwerk eine darmsaftlösliche Folie von beispielsweise 0,1 bis 1 mm Dicke ergeugt werden. Derartige Folien können auf verschiedene Weise für Arzneiformen verwendet werden. Im thermoelastischen Zustand können bei Temperaturen von 100 bis 140 Kapselteile gezogen werden. Dazu eignen sich bekannte Umformverfahren, bei denen die Formung durch mechanische Ziehstempel, durch Über- oder Unterdruck oder eine Kombination dieser Mittel bewirkt wird. Nach dem Abschneiden der ausgeformten Kapselteile kann die verbliebene Folie erneut aufgeschmolzen werden.

Formteile aus den erfindungsgemäßen Kunststoffen können auch mit anderen Formteilen aus löslichen oder unlöslichen Werkstoffen zu Dosierungseinheiten kombiniert werden, um die Auflösung bestimmter Teile des therapeutischen Systems zu steuern. Damit können begrenzte Dosen von einem oder mehreren Wirkstoffen in der Weise zu einer Dosierungseinheit zusammengefaßt werden, daß einzelne Dosen in bestimmten Abschnitten des Magen-Darm-Traktes unter definierten Bedingungen freigesetzt werden. Auch kann durch die Mitverwendung von darmsaftlöslichen Formteilen der Zerfall des Systems in kleinere, leichter ausscheidbare Teile bewirkt werden.

Dank der Form- und Schweißbarkeit der Folie bieten sich neuartige, rationelle Umhüllungsverfahren an. Man kann beispielsweise gepreßte Tablettenkerne zwischen zwei gereckten Folien unter Vakuum einsiegeln und verschweißen. Beim Erwärmen schrumpft die Hülle dicht auf den Kern auf.

Das Mischpolymerisat kann nach üblichen Verfahren der radikalischen Polymerisation hergestellt werden. Dabei können Regler, wie z.B. Alkylmercaptane, mitverwendet werden, um ein Molekulargewicht (Gewichtsmittelwert) von 50.000 bis 1.500.000 Dalton zu erreichen. Die Schmelzviskosität des isolierten Polymerisats bzw. der fertig konfektionierten Formmasse liegt vorzugsweise im Bereich von 1.000 bis 100.000 Pa s bei 120-145°C und 1-5 MPa Druck, gemessen gemäß DIN 54811, Verfahren B. Ein rationelles Verfahren ist die Polymerisation der Monomeren in Substanz, beispielsweise in einem Schneckenextruder. Vorzugsweise wird das Mischpolymerisat durch Emulsionspolymerisation in wäßriger Phase in Gegenwart von vorzugsweise anionischen Emulgatoren hergestellt und durch Sprühtrocknen, Gefriertrocknen oder durch Koagulation und Entwässerung isoliert.

Zusammen mit der Acryl- und/oder Methacrylsäure und dem Methylacrylat können gewünschtenfalls andere Alkylester der Acryl-und/oder Methacrylsäure, insbesondere solche mit 1 bis 8 Kohlenstoffatomen im Alkylrest, in Mengen bis zu 40 Gew.-% mitverwendet werden. Geeignet sind besonders Ethyl-, Propyl-, Butyl- und 2-Ethylhexylacrylat und Methyl-, Ethyl-, Propyl- und Butylmethacrylat. In untergeordneten Mengen können weitere äthylenisch ungesättigte, radikalisch polymerisierbare Monomere, wie Hydroxyalkyl(meth)acrylate, mitverwendet werden.

Das Mischpolymerisat kann in der Schmelze mit Hilfsstoffen versetzt werden, die in Arneimittelüberzugsmassen gebräuchlich sind. Dazu gehören Weichmacher, wie Zitronensäureester, Polyethylenglykole, Füllstoffe, Farbstoffe, Pigmente, Konservierungsmittel, Geschmacksstoffe, Wirkstoffe und Formtrennmittel, wie Glycerinmono- und -distearat, Gemische aus beiden sowie Stearinsäure und ihre Metallsalze.

Die konfektionierte Formmasse wird bei Temperaturen von 120 bis 180°C thermoplastisch verarbeitet. Zum Spritzgießen wird eine Viskosität unter 10.000 Pa s angestrebt, die im allgemeinen bei Temperaturen von 140 - 180°C erreichbar ist.

### BEISPIELE

### Beispiel 1

Auf einem Laborwalzwerk wurde ein durch Emulsionspolymerisation einer Monomerenmischung aus 60 Gew.-% Methylacrylat, 20 Gew.-% Methylmethacrylat und 20 Gew.-% Methacrylsäure erzeugtes und anschließend sprühgetrocknetes Copolymerisat bei einer Walzentemperatur von 160°C aufgeschmolzen. Nachdem die Schmelze homogenisiert war, wurden 6 Gew.-% Glycerinmonostearat zugegeben und mehrere Minuten gemischt. Das Walzfell wurde abgenommen, gebrochen und auf einer Schlagmühle zu einem Pulver mit einer mittleren Korngröße von 0,2 mm gemahlen.

Das Copolymerisat hat eine Schmelzviskosität von 7700 Pa s bei 145°C/5 MPa.

Zur Prüfung der thermoplastischen Verarbeitbarkeit wurde das Kunststoffpulver in die Vorkammer eines Spritzgießwerkzeugs gefüllt und bei 170°C unter einem Druck von 150 bar durch eine 0,5 mm weite Öffnung in den Formhohlraum gespritzt. Dabei wurde eine riß- und blasenfreie, leicht opake, dünnwandige Arzneimittelkapselhälfte erhalten, die nach Abkühlung aus dem geöffneten Werkzeug entnommen wurde. Sie war in einer Pufferlösung vom pH 7,5 innerhalb 90 Minuten löslich.

### Beispiele 2 bis 5

In gleicher Weise wurden sprühgetrocknete Emulsionspolymerisate der Zusammensetzungen (in Gew.-%):

| | |
|---|---|
| Beispiel 2 | Methylacrylat/Methacrylsäure 80:20 Kern/Schale-Aufbau 1:1 mit den Monomerenverhältnissen 70:30 / 90:10. |
| Beispiel 3 | Methylacrylat/Methacrylsäure 80:20 Kern/Schale-Aufbau 1:1 mit den Monomerenverhältnissen 90:10 / 70:30. |
| Beispiel 4 | Methylacrylat/Ethylacrylat/Methacrylsäure 40:25:35 |
| Beispiel 5 | Methylacrylat/Methacrylsäure 80:20 Kern/Schale-Aufbau, wobei nur der Kern unter Zusatz eines Reglers hergestellt worden war, |
| Beispiel 6 | Methylacrylat/Ethylacrylat/Butylacrylat/ Methacrylsäure 30:15:15:40 |

unter Zusatz von 2 Gew.-% Glycerinmonostearat (Beisp.4 nur 1 Gew.-%) als Entformungshilfsmittel zu Kapselhälften verarbeitet. In allen Fällen waren die Kapselhälften in Pufferlösungen vom pH 7,5 innerhalb 90 Minuten löslich.

Die Copolymerisate haben folgende Molekulargewichte M_{w} (nach der GPC-Methode, Gewichtsmittelwerte) und Schmelzviskositäten Vₛ bei 145°C/5 MPa:

| | M_{w} Dalton | Vₛ Pa s |
|---|---|---|
| Beispiel 2 | -- | 23 000 |
| Beispiel 3 | -- | 15 000 |
| Beispiel 4 | 190 000 | 192 000 |
| Beispiel 5 | 160 000 | 67 000 |
| Beispiel 6 | 200 000 | 222 000 |

### Beispiele 7 bis 9

In gleicher Weise wurden sprühgetrocknete Emulsionspolymerisate aus Methylacrylat, Methylmethacrylat und Methacrylsäure in folgenden Gewichtsverhältnissen:

| | | | |
|---|---|---|---|
| Beispiel 7 | 40 | 40 | 20 |
| Beispiel 8 | 60 | 20 | 20 |
| Beispiel 9 | 60 | 25 | 15 |

unter Zusatz von 3 Gew.-% Glycerinmonostearat als Entformungshilfsmittel zu Kapselhälften verarbeitet. Bei den Beispielen 8 und 9 ließen sich durch Einsatz von 6 bzw. 5 Gew.-% des Entformungshilfsmittels eine leichtere Entformbarkeit und glattere Kapseloberflächen erreichen. Die Kapselhälften hatten eine Länge von 16,5 mm, einen Außendurchmesser von 6,8 mm, eine Wandstärke von 0,5 mm und ein Gewicht von 146 mg.

Die Copolymerisate haben etwa folgende Schmelzviskositäten bei 145°C/5 MPa:

| | |
|---|---|
| Beispiel 7 | 85 000 Pa s |
| Beispiel 8 | 20 000 Pa s |
| Beispiel 9 | 16 000 Pa s |

### Prüfung des Auflösungsverhaltens der Kapseln gemäß Beispielen 8-9

Zwei gemäß Beispiel 8 erzeugte Steckkapselhälften wurden mit Granula einer Teilchengröße von 3 mm gefüllt und (für Versuchszwecke) mit Cyanacrylatkleber wasserdicht verschlossen. Die gefüllte Kapsel wurde zunächst in einem Becherglas mit 200 ml 0,1 n HCl als künstlichem Magensaft mit 30 Hüben pro Minute jeweils 5 cm auf und ab bewegt. Innerhalb von 2 Std. bei 37°C wurde die Kapselwand trüb; es war aber kein Zerfall zu beobachten, auch drang keine Flüssigkeit nach innen ein. Danach wurde die Flüssigkeit durch einen Phosphatpuffer pH 7,5 ersetzt und die Bewegung fortgesetzt. Der pH-Wert wurde fortlaufend gemessen und durch Zugabe von 0,5 n NaOH mittels pH-Stat konstant gehalten. Nach 30 Min. wurde die Kapsel stark trüb, gleichzeitig wurde Alkaliverbrauch beobachtet. Innerhalb der nächsten 50 Min. stieg der Alkaliverbrauch auf 0,13 ml 0,5 n NaOH an und die Kapsel öffnete sich und gab die Granula frei. Nach insgesamt 120 Min. und einem Alkaliverbrauch von 0,19 ml war die Kapsel fast vollständig aufgelöst.

Zwei gemäß Beispiel 9 erzeugte Steckkapselhälften wurden mit 60 mg Bisacodyl-Pellets, enthaltend 5 % Wirkstoff, gefüllt und mit Cyanacrylatkleber verschlossen. Die Kapsel wurde, wie oben beschrieben, 1 Std. bei 37°C in 250 ml 0,1 n HCl bewegt und zeigte dabei keine Zerfallserscheinungen. Die danach in der Flüssigkeit gemessene Extinktion von 0,002 bei 264 nm zeigt, daß höchstens 0,3 % des Wirkstoffes ausdiffundiert waren. Nach Wechsel der Behandlungsflüssigkeit zu Phosphatpuffer pH 7,5 und Anschluß eines pH-Staten setzte schon nach 5 Min. Alkaliverbrauch ein. Nach 40 Min. öffnete sich die Kapsel und gab die Pellets frei. Durch Extinktionsmessung konnte eine Wirkstofffreisetzung von 92,3 % nachgewiesen werden. Das Kapselmaterial war zu diesem Zeitpunkt etwa zur Hälfte aufgelöst.

### Untersuchungen an weiteren Copolymerisaten

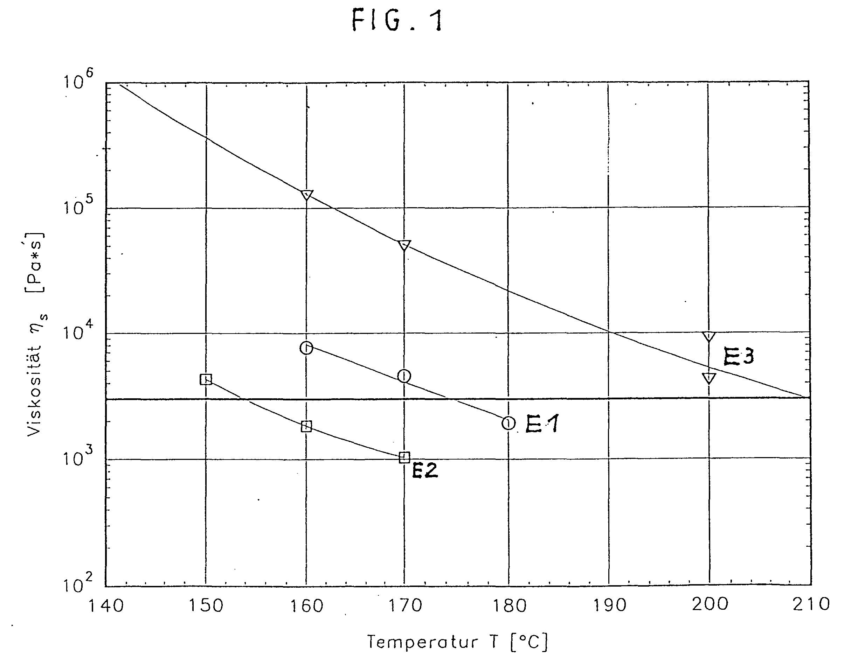

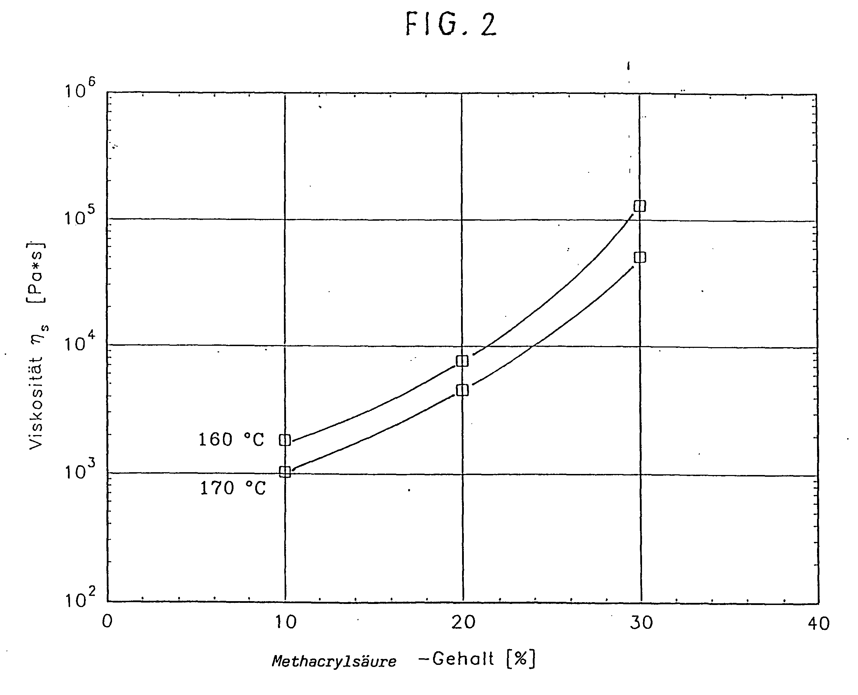

Für drei Emulsionspolymerisate E1, E2 und E3 aus Methylacrylat, Methylmethacrylat und Methacrylsäure in den Gewichtsverhältnissen:
E1: 60 : 20 : 20
E2: 65 : 25 : 10
E3: 50 : 20 : 30
wurden die Schmelzviskositäten in Abhängigkeit von der Temperatur der Polymerisatschmelze gemessen und in Figur 1 aufgetragen. Figur 2 zeigt die Abhängigkeit der Schmelzviskosität bei 160 bzw. 170°C vom Methacrylsäureanteil im Copolymerisat.

## Patentansprüche

1. Thermoplastischer Kunststoff zur Herstellung von darmsaftlöslichen Arznei-Umhüllungen, dadurch gekennzeichnet daß er aus einem Mischpolymerisat aus
A) 16 bis 40 Gew.-% Acryl- und/oder Methacrylsäure,
B) 30 bis 80 Gew.-% Methylacrylat
C) 0 bis 40 Gew.-% anderen Alkylestern der Acryl- und/oder Methacrylsäure
und gegebenenfalls üblichen Hilfsstoffen für Arzneimittelüberzügen besteht.

2. Thermoplastischer Kunststoff nach Anspruch 1, gekennzeichnet durch eine Schmelzviskosität im Bereich von *1.000* bis *100.000* Pa s bei 120 bis *145* °c.

3. Thermoplastischer Kunststoff nach Anspruch 1 oder 2, gekennzeichnet durch ein Molekulargewicht (Gewichtsmittelwert) von 50.000 bis 1.500.000 Dalton.

4. Schweißbare Kunststoff-Folie, dadurch gekennzeichnet, daß sie aus dem thermoplastischen Kunststoff nach Anspruch 1, 2 oder 3 besteht.

5. Schweißbare Kunststoff-Folie nach Anspruch 4, dadurch gekennzeichnet, daß sie ein- oder zweidimensional gereckt ist.

6. Umhüllte Arzneiform, enthaltend die thermoplastische Kunststoff-Formmasse nach Anspruch 1, 2 oder 3 als Hüllmaterial.

7. Arzneimittel-Steckkapsel, bestehend aus der thermoplastischen Kunststoff-Formmasse nach Anspruch 1, 2 oder 3.

8. Arzneimittel-Dosierungseinheit, enthaltend mehrere Wirkstoff-Dosen sowie wenigstens ein Formteil aus einem Kunststoff gemäß einem der Ansprüche 1, 2 oder 3 neben Formteilen aus anderen Werkstoffen.

9. Verwendung der thermoplastischen Kunststoff-Formmasse nach Anspruch 1, 2 oder 3 zum Umhüllen von Arzneimitteln.

10. Verfahren zur Herstellung von Arznei-Umhüllungen, dadurch gekennzeichnet, daß ein thermoplastischer Kunststoff nach Anspruch 1, 2 oder 3 geschmolzen und aus der Schmelze die Arznei-Umhüllung geformt wird.

11. Verfahren nach Anspruch 10, dadurch gekennzeichnet, daß aus der Schmelze durch Spritzgießen Arzneimittel-Steckkapseln geformt werden.

12. Verfahren zur Herstellung von Arzneimittel-Steckkapseln, dadurch gekennzeichnet, daß ein thermoplastischer Kunststoff nach Anspruch 1, 2 oder 3 geschmolzen, aus der Schmelze durch Extrusion eine Folie gebildet wird und durch thermisches Umformen der Folie Steckkapseln erzeugt werden.

## Revendications

1. Matière plastique pour la production d'enrobages pharmaceutiques entérosolubles,
caractérisée en ce qu'
elle est formée d'un polymérisat mixte à base de :
A) 16 à 40 % en poids d'acide acrylique et/ou d'acide méthacrylique,
B) 30 à 80 % en poids d'acrylate de méthyle,
C) 0 à 40 % en poids d'autres esters d'alkyle de l'acide acrylique et/ou de l'acide méthacrylique et le cas échéant de substances auxiliaires habituelles pour des revêtements de médicament.

2. Matière plastique thermoplastique selon la revendication 1,
caractérisée par
une viscosité à la fusion dans la zone de 1 000 à 100 000 Pa-s de 120 à 145°C.

3. Matière plastique thermoplastique selon la revendication 1 ou la revendication 2,
caractérisée par
un poids moléculaire (valeur moyenne de poids) allant de 50 000 à 1 500 000 daltons.

4. Feuilles de matière plastique soudable
caractérisées en ce qu'
elles consistent en une matière plastique thermoplastique selon la revendication 1, 2 ou 3.

5. Feuilles de matière plastique soudable selon la revendication 4,
caractérisées en ce qu'
elles sont étirées d'une manière uni- ou bidimensionnelle.

6. Forme pharmaceutique enrobée contenant la masse moulée en matière plastique thermoplastique selon la revendication 1, 2 ou 3 en tant que matériau d'enrobage.

7. Capsules emboîtables de médicament, formées de la masse moulée de matière plastique thermoplastique selon la revendication 1, 2 ou 3.

8. Unité de dosage de médicament contenant plusieurs doses de principe actif ainsi qu'au moins une pièce moulée à base d'une matière plastique conformément à une des revendications 1, 2 ou 3 à côté de pièces moulées à base d'autres matériaux.

9. Utilisation des masses moulées en matière plastique thermoplastique selon la revendication 1, 2 ou 3, en vue de l'enrobage de médicaments.

10. Procédé de production d'enrobages pharmaceutiques,
caractérisé en ce qu'
une matière plastique thermoplastique selon la revendication 1, 2 ou 3 est fondue et l'enrobage pharmaceutique est formé à partir du produit de fusion.

11. Procédé selon la revendication 10,
caractérisé en ce qu'
on forme des capsules emboîtées de médicament par moulage par injection à partir du produit de fusion.

12. Procédé de production de capsules emboîtées de médicament,
caractérisé en ce qu'
une matière plastique thermoplastique selon la revendication 1, 2 ou 3 est fondue, une feuille est formée par extrusion à partir du produit de fusion, et on obtient par mise en forme thermique de la feuille, des capsules emboîtables.

## Claims

1. A thermoplastic material for the production of medicament casings which are dissolvable in intestinal juices, characterised in that it comprises a copolymer of
A) 16 to 40 wt.-% acrylic and/or methacrylic acid
B) 30 to 80 wt.-% methylacrylate
C) 0 to 40 wt.-% other alkylesters of acrylic
and/or methacrylic acid and optionally usual adjuvants for medicament casings.

2. A thermoplastic material according to claim 1, characterised by a melt viscosity in the range 1,000 to 100,000 Pa s at 120 to 145° C.

3. A thermoplastic material according to claim 1 or 2, characterised by a molecular weight (weight average value) of 50,000 to 1,500,000 Daltons.

4. A weldable plastic film, characterised in that it comprises the thermoplastic material according to claim 1, 2 or 3.

5. A weldable plastic film according to claim 4, characterised in that it is stretched in one or two dimensions.

6. A coated medicament, containing the thermoplastic material according to claim 1, 2 or 3 as a casing material.

7. A medicament cachet shell comprising the thermoplastic material according to claim 1, 2 or 3.

8. A medicament dosing unit containing a plurality of doses of active ingredient and at least one moulded part of a material according to one of claims 1, 2 or 3 along with moulded parts of other materials.

9. Use of the thermoplastic material according to claim 1, 2 or 3 for encasing medicaments.

10. A process for the production of medicament casings, characterised in that a thermoplastic material according to claim 1, 2 or 3 is melted and the medicament casing is formed from the melt.

11. A process according to claim 10, characterised in that medicament cachet shells are formed from the melt by injection moulding.

12. A process for the production of medicament cachet shells, characterised in that a thermoplastic material according to claim 1, 2 or 3 is melted, a film is formed from the melt by extrusion and cachet shells are produced by thermal deformation of the film.
